(19) Europäisches Patentamt
European Patent Office
Office européen des brevets

(11) **EP 4 744 488 A1**

(12) **EUROPEAN PATENT APPLICATION**
published in accordance with Art. 153(4) EPC

(43) Date of publication:
20.05.2026 Bulletin 2026/21

(21) Application number: 24843054.8

(22) Date of filing: 11.07.2024

(51) International Patent Classification (IPC):
*A01N 31/02* (2006.01)    *A01P 1/00* (2006.01)
*A61K 8/34* (2006.01)    *A61Q 1/00* (2006.01)
*A61Q 19/00* (2006.01)

(52) Cooperative Patent Classification (CPC):
A01N 31/02; A01P 1/00; A61K 8/34; A61Q 1/00;
A61Q 19/00

(86) International application number:
PCT/JP2024/025113

(87) International publication number:
WO 2025/018263 (23.01.2025 Gazette 2025/04)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 14.07.2023 JP 2023115998

(71) Applicant: IBIDEN CO., LTD.
Ogaki-shi, Gifu 503-8604 (JP)

(72) Inventors:
• HATTORI, Saki
  Ogaki-shi, Gifu 503-8503 (JP)

• FURUICHI, Wataru
  Ogaki-shi, Gifu 503-8503 (JP)
• YOKOTA, Teruaki
  Ogaki-shi, Gifu 503-8503 (JP)
• MIZUMUKAI, Yuki
  Ogaki-shi, Gifu 503-8503 (JP)
• OHNO, Katsuya
  Ogaki-shi, Gifu 503-8503 (JP)

(74) Representative: Hoefer & Partner Patentanwälte
mbB
Pilgersheimer Straße 20
81543 München (DE)

(54) **COMPOSITION AND STARTING MATERIAL COMPOSITION OF COSMETIC**

(57) Provided is a composition capable of appropriately exhibiting a viral proliferation inhibitory effect. The composition of the present invention contains at least (2R,3R)-2,3-butanediol and/or (2S,3S)-2,3-butanediol and (meso)-2,3-butanediol, wherein in terms of weight ratio, an amount of the (meso)-2,3-butanediol is equal to or less than twice a combined amount of the (2R,3R)-2,3-butanediol and the (2S,3S)-2,3-butanediol.

EP 4 744 488 A1

# FIG.1

Ion 29.00(29.00~800.00) : 2.D¥data.ms

(meso)-2,3-Butanediol

(2R,3R)-2,3-Butanediol

(2S,3S)-2,3-Butanediol

Abundance

190000
180000
170000
160000
150000
140000
130000
120000
110000
100000
90000
80000
70000
60000
50000
40000
30000
20000
10000
0

0 1 2 3 4 5 6 7 8 9 10 11 12 13 14 15 16 17 18 19 20 21 22 23 24 25 26 27 28 29

Time

**Description**

TECHNICAL FIELD

The present invention relates to compositions and cosmetic raw material compositions.

BACKGROUND ART

2,3-Butanediol (hereinafter, also referred to as "2,3-BDO") is used, for example, as an antifreeze solution, and as a raw material for production of methyl ethyl ketone and 1,3-butadiene via dehydration. Also, 2,3-BDO is a chemical substance with significant potential industrial applications, including use as a liquid fuel having a calorific value of 27198 kJ.kg$^{-1}$, which is comparable to those of methanol (22081 kJ.kg$^{-1}$) and ethanol (29055 kJ.kg$^{-1}$). Other potential applications include its use in printing inks, perfumes, cosmetic raw materials, fumigants, humidifiers, and softeners, the production of explosives and plasticizers, and the production of carriers for pharmaceutical products.

The application of 2,3-BDO as an antibacterial and antiviral agent has also been studied in recent years. Patent Literature 1 discloses an antiviral composition consisting of 2,3-butanediol and an acid. In this document, 2,3-butanediol itself is disclosed as an alcohol which cannot inactivate viruses.

Patent Literature 2 discloses an antibacterial agent composed of (meso)-2,3-butanediol, but does not mention antiviral activity.

Patent Literatures 1 and 2 and Non-Patent Literature 1 are all incorporated herein by reference.

CITATION LIST

- Patent Literature

Patent Literature 1: JP 2018 -531991 T
Patent Literature 2: JP 2017 -531671 T

- Non-Patent Literature

Non-Patent Literature 1: Reports of the Central Customs Laboratory, No. 20, 1980, pp.123 to 127

SUMMARY OF INVENTION

- Technical Problem

As described above, 2,3-BDO has not been conventionally recognized as having an antiviral activity, and its use as an agent for inhibiting viral proliferation has not been examined at all.

An object of the present invention is to provide a composition capable of appropriately exhibiting a viral proliferation inhibitory effect in applications of 2,3-BDO in which a viral proliferation inhibitory effect is required.

- Solution to Problem

As a result of intensive research conducted by the present inventors to investigate the reason why 2,3-BDO does not exhibit inhibitory activity against viral proliferation as described in Patent Literature 1, the inventors have newly found that there is a correlation between the ratio of optical isomers of 2,3-BDO and inhibitory activity against viruses.

Generally, a chemically synthesized product of 2,3-BDO contains three optical isomers, namely (2R,3R)-2,3-butanediol, (2S,3S)-2,3-butanediol, and (meso)-2,3-butanediol.

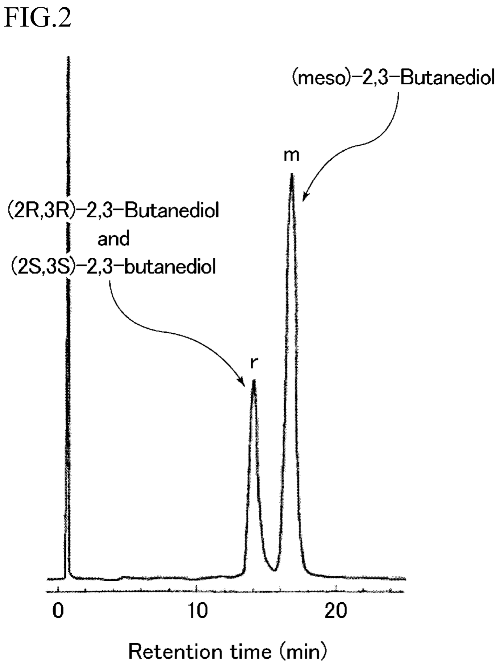

FIG. 2 is a gas chromatogram showing the ratio of optical isomers of 2,3-BDO, as described in Non-Patent Literature 1.

As shown in FIG. 2, a typical chemically synthesized product of 2,3-BDO contains the optical isomers in a weight ratio of (2R,3R)-2,3-butanediol : (2S,3S)-2,3-butanediol : (meso)-2,3-butanediol = 1:1:4.25 (the peak observed around 14 minutes corresponds to both (2R,3R)-2,3-butanediol and (2S,3S)-2,3-butanediol (indicated by the initial letter "r" for racemic mixture in FIG. 2) while the peak around 16 minutes corresponds to (meso)-2,3-butanediol (indicated by "m" in FIG. 2)).

The present inventors have found that (meso)-2,3-butanediol promotes viral proliferation while (2R,3R)-2,3-butanediol and (2S,3S)-2,3-butanediol inhibit viral proliferation.

A typical chemically synthesized product of 2,3-BDO is not found to exhibit antiviral activity presumably because the chemically synthesized product of 2,3-BDO contains both (meso)-2,3-butanediol, which promotes viral proliferation, and (2R,3R)-2,3-butanediol and (2S,3S)-2,3-butanediol, which inhibit viral proliferation. The coexistence of these isomers seemingly results in antagonistic effects, thereby leading to minimal observable antiviral activity.

Herein, the term "viral proliferation inhibitory effect" refers to a condition in which the viral proliferation capacity is reduced upon administration of the subject composition, as compared to administration of water. In such cases, the subject composition is considered to exhibit a viral proliferation inhibitory effect.

The present inventors have found that viral proliferation can be sufficiently inhibited by adjusting the weight ratio of (meso)-2,3-butanediol, (2R,3R)-2,3-butanediol, and (2S,3S)-2,3-butanediol.

In other words, the composition of the present invention contains at least (2R,3R)-2,3-butanediol and/or (2S,3S)-2,3-butanediol and (meso)-2,3-butanediol, wherein in terms of weight ratio, the amount of (meso)-2,3-butanediol is equal to or less than twice the combined amount of (2R,3R)-2,3-butanediol and (2S,3S)-2,3-butanediol.

In terms of weight ratio, the amount of (meso)-2,3-butanediol is more preferably equal to or less than 1.5 times, still more preferably equal to or less than 0.5 times, even more preferably equal to or less than 0.13 times, particularly preferably equal to or less than 0.05 times, the combined amount of (2R,3R)-2,3-butanediol and (2S,3 S)-2,3-butanediol.

When the optical isomers of 2,3-BDO are present in the above ratio in the composition, a suitable viral proliferation inhibitory effect is exhibited.

Preferably, the composition of the present invention contains (2S,3S)-2,3-butanediol, and the amount of (2S,3S)-2,3-butanediol is greater than the amount of (meso)-2,3-butanediol.

Also preferably, the composition of the present invention contains (2R,3R)-2,3-butanediol, and the amount of (2R,3R)-2,3-butanediol is greater than the amount of (meso)-2,3-butanediol.

When the optical isomers of 2,3-BDO are present in the above ratio in the composition, a more suitable viral proliferation inhibitory effect is exhibited.

More preferably, the composition of the present invention contains both (2S,3S)-2,3-butanediol and (2R,3R)-2,3-butanediol, and the amount of (2S,3S)-2,3-butanediol is greater than the amount of (2R,3R)-2,3-butanediol and greater than the amount of (meso)-2,3-butanediol.

A comparison between (2S,3S)-2,3-butanediol and (2R,3R)-2,3-butanediol shows that (2S,3S)-2,3-butanediol exhibits a higher viral proliferation inhibitory effect.

Therefore, with the amount of (2S,3S)-2,3-butanediol set to fall within the above range, a more suitable viral proliferation inhibitory effect is exhibited.

The composition of the present invention may contain both (2S,3S)-2,3-butanediol and (2R,3R)-2,3-butanediol, and the amount of (2R,3R)-2,3-butanediol may be greater than the amount of (2S,3S)-2,3-butanediol and greater than the amount of (meso)-2,3-butanediol.

A comparison between (2S,3S)-2,3-butanediol and (2R,3R)-2,3-butanediol shows that (2R,3R)-2,3-butanediol is colorless and transparent, while (2S,3S)-2,3-butanediol appears slightly yellow. Therefore, from the viewpoint of color adjustability, the amount of (2R,3R)-2,3-butanediol is preferably greater than the amount of (2S,3S)-2,3-butanediol in the composition of the present invention.

The composition of the present invention may be free from (2S,3S)-2,3-butanediol.

As described above, (2R,3R)-2,3-butanediol is colorless and transparent, while (2S,3S)-2,3-butanediol appears slightly yellow. Therefore, from the viewpoint of color adjustability, the composition of the present invention is preferably free from (2S,3S)-2,3-butanediol.

The composition of the present invention can inhibit viral proliferation even without (2S,3S)-2,3-butanediol.

Herein, the expression "the composition is free from (2S,3S)-2,3-butanediol" refers to a state where no peak with S/N = 3 or higher is observed at the retention time corresponding to (2S,3S)-2,3-butanediol (around 12.8 minutes in each of the examples and the comparative examples) in the GC-MS analysis of the composition under the above conditions.

The retention time corresponding to (2S,3S)-2,3-butanediol can be identified using a standard sample.

The composition of the present invention is preferably free from a racemic mixture of (2R,3R)-2,3-butanediol and (2S,3S)-2,3-butanediol.

This is because the antiviral activities of (2R,3R)-2,3-butanediol and (2S,3S)-2,3-butanediol tend to decrease when they are present as a racemic mixture.

The composition of the present invention is preferably used as a viral proliferation inhibitor.

As described above, the composition of the present invention exhibits a viral proliferation inhibitory effect. Therefore, the composition of the present invention is suitable as a viral proliferation inhibitor.

The cosmetic raw material composition of the present invention contains the composition of the present invention.

As described above, the composition of the present invention exhibits a viral proliferation inhibitory effect. Therefore, when a cosmetic product is produced from a cosmetic raw material composition containing the composition of the present invention, the cosmetic product exhibits a viral inhibitory effect.

- Advantageous Effects of Invention

The present invention can provide a composition capable of appropriately exhibiting a viral proliferation inhibitory effect.

BRIEF DESCRIPTION OF DRAWINGS

FIG. 1 is a chromatogram showing the results of GC-MS analysis of a composition according to Example 5.
FIG. 2 is a gas chromatogram showing the ratio of optical isomers of 2,3-BDO, as described in Non-Patent Literature 1.

DESCRIPTION OF EMBODIMENTS

(Detailed Description of the Invention)

The present invention relates to a composition containing at least (2R,3R)-2,3-butanediol and/or (2S,3S)-2,3-butanediol and (meso)-2,3-butanediol, wherein in terms of weight ratio, the amount of (meso)-2,3-butanediol is equal to or less than twice the combined amount of (2R,3R)-2,3-butanediol and (2S,3S)-2,3-butanediol.
(2R,3R)-2,3-Butanediol and (2S,3S)-2,3-butanediol have excellent viral proliferation inhibitory effects. In contrast, (meso)-2,3-butanediol causes viral proliferation.
Since commercially available 2,3-BDO contains these three optical isomers, its viral proliferation inhibitory effect is hardly observed.
However, in the present invention, the viral proliferation inhibitory effect is expressed since the amount of (meso)-2,3-butanediol is equal to or less than twice the combined amount of (2R,3R)-2,3-butanediol and (2S,3S)-2,3-butanediol in terms of weight ratio.
The composition of the present invention exhibits a viral proliferation inhibitory effect against enveloped viruses and non-enveloped viruses.
Furthermore, since the composition of the present invention contains (meso)-2,3-butanediol, the composition can exhibit high moisturizing and moisture-absorbing effects, and can simultaneously exhibit both a viral proliferation inhibitory effect and moisturizing and moisture-absorbing effects (see Chemical Abstracts Service, CAS: 5341-95-7).
When the composition of the present invention is free from either (2R,3R)-2,3-butanediol or (2S,3S)-2,3-butanediol, the term "combined amount of (2R,3R)-2,3-butanediol and (2S,3S)-2,3-butanediol" means the amount of either (2R,3R)-2,3-butanediol or (2S,3S)-2,3-butanediol contained in the composition.
A comparison between (2S,3S)-2,3-butanediol and (2R,3R)-2,3-butanediol shows that (2S,3S)-2,3-butanediol exhibits a higher viral proliferation inhibitory effect. Therefore, from the viewpoint of a viral proliferation inhibitory effect, the amount of (2S,3S)-2,3-butanediol is preferably greater than the amount of (2R,3R)-2,3-butanediol in the composition of the present invention.
Further, a comparison between (2S,3S)-2,3-butanediol and (2R,3R)-2,3-butanediol shows that (2R,3R)-2,3-butanediol is colorless and transparent, while (2S,3S)-2,3-butanediol appears slightly yellow. Therefore, from the viewpoint of color adjustability, the amount of (2R,3R)-2,3-butanediol is preferably greater than the amount of (2S,3S)-2,3-butanediol in the composition of the present invention.
In the composition of the present invention, in terms of weight ratio, the amount of (meso)-2,3-butanediol is preferably equal to or less than 1.5 times, more preferably equal to or less than 0.5 times, still more preferably equal to or less than 0.13 times, particularly preferably equal to or less than 0.05 times, the combined amount of (2R,3R)-2,3-butanediol and (2S,3S)-2,3-butanediol. This is because the viral proliferation inhibitory effect is significant.
The composition of the present invention may also contain both (2R,3R)-2,3-butanediol and (2S,3S)-2,3-butanediol, and the weight ratio of the amount of (2R,3R)-2,3-butanediol/the amount of (2S,3S)-2,3-butanediol may be 1/1000 to 1000/1. The weight ratio of the amount of (2R,3R)-2,3-butanediol/the amount of (2S,3S)-2,3-butanediol may be 1/100 to 100/1. The weight ratio of the amount of (2R,3R)-2,3-butanediol/the amount of (2S,3S)-2,3-butanediol may also be 1/10 to 10/1.
The composition of the present invention containing (meso)-2,3-butanediol is preferably free from a racemic mixture containing (2R,3R)-2,3-butanediol and (2S,3S)-2,3-butanediol in equal amounts. This is because the antiviral activities of (2R,3R)-2,3-butanediol and (2S,3S)-2,3-butanediol tend to decrease when they are present as a racemic mixture.
Preferably, the composition of the present invention contains (2S,3S)-2,3-butanediol, and the amount of (2S,3S)-2,3-butanediol is greater than the amount of (meso)-2,3-butanediol.

More preferably, the composition of the present invention contains both (2S,3S)-2,3-butanediol and (2R,3R)-2,3-butanediol, and the amount of (2S,3S)-2,3-butanediol is greater than the amount of (2R,3R)-2,3-butanediol and greater than the amount of (meso)-2,3-butanediol.

As described above, (2S,3S)-2,3-butanediol exhibits a higher viral proliferation inhibitory effect than (2R,3R)-2,3-butanediol.

Therefore, with the amount of (2S,3S)-2,3-butanediol set to fall within the above range, a more suitable viral proliferation inhibitory effect is exhibited.

Also preferably, the composition of the present invention contains (2R,3R)-2,3-butanediol, and the amount of (2R,3R)-2,3-butanediol is greater than the amount of (meso)-2,3-butanediol.

More preferably, the composition of the present invention contains both (2S,3S)-2,3-butanediol and (2R,3R)-2,3-butanediol, and the amount of (2R,3R)-2,3-butanediol is greater than the amount of (2S,3S)-2,3-butanediol and greater than the amount of (meso)-2,3-butanediol.

(2R,3R)-2,3-Butanediol is colorless and transparent, while (2S,3S)-2,3-butanediol appears slightly yellow. Therefore, the amount of (2R,3R)-2,3-butanediol is preferably greater than the amount of (2S,3S)-2,3-butanediol, as this allows for higher color adjustability of the composition. This is particularly advantageous when the composition of the present invention is used as a cosmetic raw material since the color adjustability of the cosmetic product can be ensured.

The composition of the present invention may be free from (2S,3S)-2,3-butanediol from the viewpoint of the color adjustability of the cosmetic product. The composition of the present invention can inhibit viral proliferation even without (2S,3S)-2,3-butanediol.

In the composition of the present invention, the combined amount of (2R,3R)-2,3-butanediol and/or (2S,3S)-2,3-butanediol and (meso)-2,3-butanediol is preferably 5% by weight to 100% by weight relative to the weight of the whole composition.

The composition of the present invention can be used without limitation as a viral proliferation inhibitor capable of exhibiting a viral proliferation inhibitory effect.

Specifically, the composition of the present invention can be used as a topical skin preparation, a cosmetic composition, a pharmaceutical composition, or a food additive, for example. Furthermore, the composition of the present invention can be formulated, for example, in the form of makeup cosmetics, toners, creams, lotions, essences, or facial masks, and more specifically, in the form of softening lotions, astringent lotions, nourishing lotions, eye creams, nourishing creams, massage creams, cleansing creams, cleansing foams, cleansing waters, powders, foundations, makeup bases, essences, or facial masks. The form is not particularly limited. In each of these forms, other components may be appropriately selected and blended by those skilled in the art, depending on the type of composition or intended use, without undue difficulty.

The composition of the present invention can also preferably be formulated as a cleansing agent composition. Although the form thereof is not particularly limited, the composition may specifically be formulated in the form of, for example, hand sanitizers, hand washes, body washes, cleansing creams, cleansing gels, cleansing foams, cleansing waters, soaps, or wet wipes.

In addition, the composition of the present invention may be sprayed onto areas where a viral proliferation inhibitory effect is required, or may be used to impregnate wet wipes or the like.

The cosmetic raw material composition of the present invention contains the composition of the present invention.

As described above, the composition of the present invention exhibits a viral proliferation inhibitory effect. Therefore, when a cosmetic product is produced from a cosmetic raw material composition containing the composition of the present invention, the cosmetic product exhibits a viral proliferation inhibitory effect.

The composition of the present invention is preferably stored or transported in a container sealed with an inert gas or with air having a humidity of 10% or less (including 0%). This is because such storage or transportation can prevent moisture absorption and thereby inhibit deterioration of the composition, such as oxidation. Examples of the inert gas include nitrogen, argon, helium, and carbon dioxide.

Next, a method for producing the composition of the present invention will be described. The composition of the present invention can be prepared by mixing industrially produced (meso)-2,3-butanediol, (2R,3R)-2,3-butanediol, and (2S,3S)-2,3-butanediol in a predetermined ratio.

The optical isomers of 2,3-BDO can also be produced using microorganisms. For example, such methods are described in JP H10-234390 A (bacteria belonging to the genus *Bacillus*), JP 2015-518736 T (genetically engineered *Escherichia coli*), and JP 2004-357639 A (genetically engineered *Escherichia coli*).

(meso)-2,3-Butanediol, (2R,3R)-2,3-butanediol, and (2S,3S)-2,3-butanediol are commercially available as, specifically, (meso)-2,3-butanediol (available from Sigma-Aldrich, product No. 361461), (2R,3R)-2,3-butanediol (available from Tokyo Chemical Industry Co., Ltd. (TCI), product No. B1161), and (2S,3S)-2,3-butanediol (available from Tokyo Chemical Industry Co., Ltd. (TCI), product No. B1343), respectively.

JP H10-234390 A, JP 2015-518736 T, and JP 2004-357639 A are incorporated herein by reference.

(Method for Measuring Proportions of Optical Isomers of 2,3-BDO in Composition)

In the composition of the present invention, the proportions of (meso)-2,3-butanediol, (2R,3R)-2,3-butanediol, and (2S,3S)-2,3-butanediol can be quantitatively and qualitatively analyzed by gas chromatography-mass spectrum (GC-MS) analysis using a chiral column. The separation technique for the three optical isomers of 2,3-BDO is owned by Agilent Technologies, Inc. For example, the three optical isomers of 2,3-BDO can be separated under the analytical conditions described below. Furthermore, quantitative analysis can be performed by preparing a calibration curve using respective standard samples of the optical isomers.

Apparatus: GC/MS: 7890A/5975C available from Agilent Technologies, Inc.
Measurement conditions:

Column: CP-Chirasil, 25 m × 0.25 mm i.d. × 0.25 μm film thickness (Agilent Technologies, Inc.)
Column temperature: 80°C (5 min) → +2°C/min → 150°C
Column pressure: 51.5 kPa (constant linear velocity mode)
Carrier gas: He (1.0 mL/min)
Injection port: 200°C, split ratio 10:1
Injection volume: 1 μL
Detector: MS
Ionization method: EI method
Electron energy: 70 eV
EM voltage: 1294 V
Ion source temperature: 230°C
Interface temperature: 200°C

Mass range: m/z = 10 to 800

The following matters are described herein.

Disclosure (1) relates to a composition containing at least (2R,3R)-2,3-butanediol and/or (2S,3S)-2,3-butanediol and (meso)-2,3-butanediol, wherein in terms of weight ratio, an amount of the (meso)-2,3-butanediol is equal to or less than twice a combined amount of the (2R,3R)-2,3-butanediol and the (2S,3S)-2,3-butanediol.

Disclosure (2) relates to the composition according to disclosure (1), wherein in terms of weight ratio, the amount of the (meso)-2,3-butanediol is equal to or less than 1.5 times the combined amount of the (2R,3R)-2,3-butanediol and the (2S,3S)-2,3-butanediol.

Disclosure (3) relates to the composition according to disclosure (1), wherein in terms of weight ratio, the amount of the (meso)-2,3-butanediol is equal to or less than 0.5 times the combined amount of the (2R,3R)-2,3-butanediol and the (2S,3S)-2,3-butanediol.

Disclosure (4) relates to the composition according to disclosure (1), wherein in terms of weight ratio, the amount of the (meso)-2,3-butanediol is equal to or less than 0.13 times the combined amount of the (2R,3R)-2,3-butanediol and the (2S,3S)-2,3-butanediol.

Disclosure (5) relates to the composition according to disclosure (1), wherein in terms of weight ratio, the amount of the (meso)-2,3-butanediol is equal to or less than 0.05 times the combined amount of the (2R,3R)-2,3-butanediol and the (2S,3S)-2,3-butanediol.

Disclosure (6) relates to the composition according to any one of disclosures (1) to (5), wherein the composition contains the (2S,3S)-2,3-butanediol, and the amount of the (2S,3S)-2,3-butanediol is greater than the amount of the (meso)-2,3-butanediol.

Disclosure (7) relates to the composition according to any one of disclosures (1) to (5), wherein the composition contains the (2R,3R)-2,3-butanediol, and the amount of the (2R,3R)-2,3-butanediol is greater than the amount of the (meso)-2,3-butanediol.

Disclosure (8) relates to the composition according to any one of disclosures (1) to (7), wherein the composition contains both the (2S,3S)-2,3-butanediol and the (2R,3R)-2,3-butanediol, and the amount of the (2S,3S)-2,3-butanediol is greater than the amount of the (2R,3R)-2,3-butanediol and greater than the amount of the (meso)-2,3-butanediol.

Disclosure (9) relates to the composition according to any one of disclosures (1) to (7), wherein the composition contains both the (2S,3S)-2,3-butanediol and the (2R,3R)-2,3-butanediol, and the amount of the (2R,3R)-2,3-butanediol is greater than the amount of the (2S,3S)-2,3-butanediol and greater than the amount of the (meso)-2,3-butanediol.

Disclosure (10) relates to the composition according to any one of disclosures (1) to (5) and (7), wherein the composition is free from (2S,3S)-2,3-butanediol.

Disclosure (11) relates to the composition according to any one of disclosures (1) to (7), wherein the composition is free from a racemic mixture of (2R,3R)-2,3-butanediol and (2S,3S)-2,3-butanediol.

Disclosure (12) relates to the composition according to any one of disclosures (1) to (11), for use as a viral proliferation inhibitor.

Disclosure (13) relates to a cosmetic raw material composition, containing the composition according to any one of disclosures (1) to (12).

EXAMPLES

(Examples 1 to 8) and (Comparative Examples 1 to 3)

Compositions according to Examples 1 to 8 were each prepared by mixing (meso)-2,3-butanediol (available from Sigma-Aldrich, product No. 361461), (2R,3R)-2,3-butanediol (available from Tokyo Chemical Industry Co., Ltd., product No. B1161), (2S,3S)-2,3-butanediol (available from Tokyo Chemical Industry Co., Ltd., product No. B1343), and water (ion-exchanged water) in the corresponding weight ratio shown in Table 1.

The composition according to Comparative Example 1 consists only of water, and the composition according to Comparative Example 2 contains a mixture of (meso)-2,3-butanediol and water.

Furthermore, the composition according to Comparative Example 3 contains (2R,3R)-2,3-butanediol, (2S,3S)-2,3-butanediol, (meso)-2,3-butanediol, and water, wherein the weight ratio of (2R,3R)-2,3-butanediol : (2S,3S)-2,3-butanediol : (meso)-2,3-butanediol is 1:1:4.25.

The composition according to Example 5 was subjected to the GC-MS analysis described in the "(Method for Measuring Proportions of Optical Isomers of 2,3-BDO in Composition)" above.

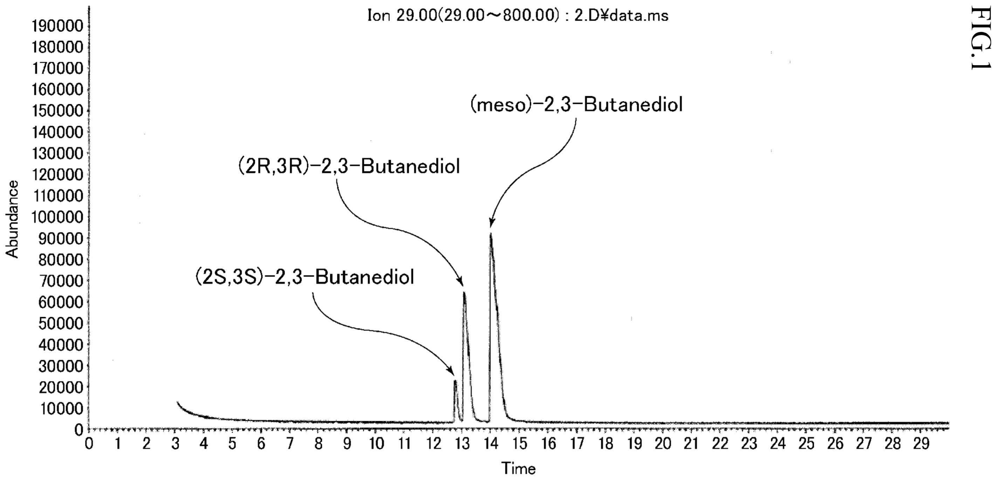

FIG. 1 is a chromatogram showing the results of GC-MS analysis of the composition according to Example 5.

FIG. 1 demonstrates that (meso)-2,3-butanediol, (2R,3R)-2,3-butanediol, and (2S,3S)-2,3-butanediol can be separated even when they are present together in a composition.

FIG. 1 also indicates that the weight ratio of the amounts of (meso)-2,3-butanediol, (2R,3R)-2,3-butanediol, and (2S,3S)-2,3-butanediol can be calculated based on the chromatogram.

(Determination of the Number of Residual Phage Viruses)

The viral proliferation inhibition effect of each of the compositions according to the examples and comparative examples was evaluated by determining the residual number of phage viruses in accordance with JIS R 1756.

(1) 10 g of tryptophan, 5 g of yeast extract, 10 g of sodium chloride, and 15 g of agar were mixed in 1 L of water and autoclaved at 120°C for 10 minutes. The resulting solution was poured onto a Petri dish (90 mm φ × 15 mm), cooled, and allowed to solidify to prepare a 1.5% LB agar medium.

(2) A 300 μL portion of each of the compositions according to the examples and comparative examples was mixed with 300 μL of bacteriophage Qβ ($10^8$ cpu/ml). The resulting mixture was stirred by inverting 3 times, and then left to stand at room temperature for 4 hours.

(3) A 200 μL portion of the mixture obtained in step (2) was mixed with 9.8 mL of SCDLP solution. The SCDLP solution was prepared by dissolving "Nutrient Broth" (available from FUJIFILM Wako Pure Chemical Corporation) in water to achieve a concentration of 41.8 g/L, followed by autoclaving at 121°C and 2 atm for 20 minutes.

(4) A 1 mL portion of the mixture obtained in step (3) was added to a test tube containing *Escherichia coli* cells (100 μL).

(5) To 1 mL of the mixture obtained in step (4), 4 mL of a 0.5% LB agar solution was added, and the mixture was stirred by inverting once. The LB agar solution was prepared by mixing 10 g of tryptophan, 5 g of yeast extract, 10 g of sodium chloride, and 15 g of agar with 1 L of water and keeping the mixture at 45°C.

(6) The mixture obtained in step (5) was poured onto the LB agar medium in the Petri dish prepared in step (1), and spread over the surface of the agar medium by slowly rotating the Petri dish.

(7) The mixture was then left to stand at 37°C for 16 hours.

(8) The number of plaques that appeared in the Petri dish was counted visually.

The results are shown in Table 1.

[Table 1]

| | Weight ratio of optical isomers | | | Total weight proportion (weight ratio) of optical isomers of 2,3-butanediol in composition | The number of plaques | Virus inactivation level |
|---|---|---|---|---|---|---|
| | (meso)-2,3-Butanediol | (2R,3-R)-2,3-Butanediol | (2S,3S)-2,3-Butanediol | | | |
| Example 1 | 4 | 5 | 1 | 0.5 | 2 | -2.76 |
| Example 2 | 4 | 10 | 1 | 0.5 | 0 | -2.50 |
| Example 3 | 4 | 1 | 5 | 0.5 | 0 | -4.92 |
| Example 4 | 4 | 1 | 10 | 0.5 | 0 | -4.92 |
| Example 5 | 10 | 5.6 | 1 | 0.5 | 4 | -2.15 |
| Example 6 | 4 | 1 | 1 | 0.5 | 21 | -2.02 |
| Example 7 | 1 | 7.8 | 0 | 0.5 | 13 | -2.08 |
| Example 8 | 1 | 20.5 | 0 | 0.5 | 9 | -2.12 |
| Comparative Example 1 | 0 | 0 | 0 | 0 (Water alone) | 51 | -1.20 |
| Comparative Example 2 | 4 | 0 | 0 | 0.25 | 74 | -1.05 |
| Comparative Example 3 | 4.25 | 1 | 1 | 0.5 | 55 | -1.15 |

The plaques that appeared in the Petri dish in step (8) will be described.

By performing step (7), *Escherichia coli* proliferates, forming a bacterial lawn of *Escherichia coli* across the entire surface of the LB agar medium in the Petri dish. If an active bacteriophage (virus) remains in the mixture obtained in step (5), it induces lysis of *Escherichia coli* cell membranes, causing *Escherichia coli* to disappear. In these areas, no bacterial lawn of *Escherichia coli* will form, leaving only the LB agar medium exposed. Such an area with only the LB agar medium, formed by the disappearance of *Escherichia coli,* is called plaque. By counting the number of plaques, the number of remaining bacteriophages (viruses) can be counted.

A large number of such plaques indicates a large number of active bacteriophages (viruses), while a small number of plaques indicates a small number of active bacteriophages (viruses).

In this experiment, a small number of plaques means that the bacteriophage (virus) activity was inhibited by the composition according to one of the examples and comparative examples used in step (2).

In other words, a small number of observed plaques means that the composition used in step (2) has a high bacteriophage (viral) proliferation inhibitory effect.

(Antiviral Evaluation Using Bacteriophage)

To evaluate the viral proliferation inhibitory effect (hereinafter, referred to as "antiviral" activity) of the compositions according to Examples 1 to 8 and Comparative Examples 1 to 3, the degree of bacteriophage inactivation was measured using a modified method based on JIS R1756, Determination of antiviral activity of photocatalytic materials under indoor lighting environment.

In other words, the compositions according to Examples 1 to 8, water (Comparative Example 1), the aqueous solution of (meso)-2,3-butanediol (Comparative Example 2), and the composition of Comparative Example 3 were individually mixed with bacteriophage Qβ in respective sample tubes, stirred by inverting, and then left to stand for 4 hours to inactivate the bacteriophage (virus). Thereafter, the bacteriophage was caused to infect *Escherichia coli,* and the sample was left to stand overnight to measure the number of infectious bacteriophages (viruses).

The measurement results are each expressed as the degree of bacteriophage (virus) inactivation, based on the concentration of bacteriophage (virus) that lost infectivity to *Escherichia coli.* Here, the concentration of bacteriophage (virus) that lost infectivity to *Escherichia coli* (bacteriophage (virus) inactivation level) was used as an indicator of bacteriophage (virus) concentration. The degree of bacteriophage (virus) inactivation of the Examples was calculated based on the bacteriophage (virus) inactivation level.

The bacteriophage (virus) inactivation level refers to the result of calculating the concentration of bacteriophage (virus) that lost infectivity to *Escherichia coli,* based on the measurement of the concentration of bacteriophage particles capable of infecting *Escherichia coli* in an antiviral test using bacteriophage Qβ at a concentration of 8.3 million particles/mL. In other words, the bacteriophage (virus) inactivation level refers to the concentration of bacteriophage particles incapable of infecting *Escherichia coli* relative to the bacteriophage Qβ concentration, and can be calculated by the formula: (bacteriophage Qβ concentration - concentration of bacteriophage (virus) particles capable of infecting *Escherichia coli*)/(bacteriophage Qβ concentration) $\times$ 100.

The bacteriophage (virus) inactivation level is used to calculate the degree of bacteriophage (virus) inactivation.

The degree of bacteriophage (virus) inactivation refers to a numerical value (expressed as a negative value) represented by the common logarithm log(1 - X), where the initial amount of bacteriophage (virus) is defined as 1 and X represents the relative amount of bacteriophage (virus) that lost infectivity after the bacteriophage (virus) inactivation treatment. A larger absolute value indicates a higher bacteriophage (virus) inactivation capability. For example, if 99.9% of the initial bacteriophage (virus) is inactivated, the degree of bacteriophage (virus) inactivation is expressed by log(1 - 0.999) = -3.00. The ratio of the amount of bacteriophage (virus) that lost infectivity after the bacteriophage (virus) inactivation treatment to the total amount of bacteriophage (virus) before the bacteriophage (virus) inactivation treatment, expressed as a percentage (99.9% in the above case), is called the bacteriophage (virus) inactivation level. As described above, the degree of bacteriophage (virus) inactivation was determined based on the bacteriophage (virus) inactivation level. Table 1 shows the results.

The degree of virus inactivation herein was evaluated in accordance with JIS Z 2801 and JIS L 1922.

The degree of virus inactivation has the opposite sign to the antiviral activity value described below. According to JIS L 1922, antiviral activity is considered present when the antiviral activity value is 2.0 or higher, and is considered sufficient when the antiviral activity value is 3.0 or higher (see JP 2018-177747 A incorporated herein by reference).

The results of Table 1 demonstrate that in a composition in which the amount of (meso)-2,3-butanediol is equal to or less than 1.5 times, particularly equal to or less than 0.5 times, the combined amount of (2R,3R)-2,3-butanediol and (2S,3S)-2,3-butanediol in terms of weight ratio, the number of plaques observed is 0 or nearly 0, indicating a high viral proliferation inhibitory activity.

In Comparative Example 2 using the mixture composition of (meso)-2,3-butanediol and water, the number of plaques is greater than that in water alone in Comparative Example 1, indicating the viral proliferation effect of (meso)-2,3-butanediol. Therefore, these test results support the understanding of why 2,3-butanediol that is industrially produced and sold as a reagent (with a composition ratio of ((2R,3R)-2,3-butanediol : (2S,3S)-2,3-butanediol : (meso)-2,3-butanediol =1:1:4.25) does not exhibit a viral proliferation inhibitory effect.

The reason for the phenomenon in which viral proliferation is promoted or inhibited depending on the optical isomers of 2,3-BDO is presumed to be as follows.

First, based on the comparison between Comparative Example 1 and Comparative Example 2, (meso)-2,3-butanediol is predicted to be a substance that facilitates viral proliferation, likely by promoting the production of a certain substance biosynthesized during viral proliferation.

On the other hand, (2R,3R)-2,3-butanediol and (2S,3S)-2,3-butanediol are predicted to act as antagonists to (meso)-2,3-butanediol, inhibiting the production of the substance biosynthesized during viral proliferation.

In other words, when (2R,3R)-2,3-butanediol and (2S,3S)-2,3-butanediol are present, the substance biosynthesized during viral proliferation is not produced, resulting in the suppression of the virus proliferation. Consequently, the virus can be inactivated.

This is also supported by the results of Comparative Example 3. In Comparative Example 3, the amount of (meso)-2,3-butanediol is 2.1 times the combined amount of (2R,3R)-2,3-butanediol and (2S,3S)-2,3-butanediol. The viral proliferation inhibitory effect is lower than that of water in Comparative Example 2, as indicated by the number of plaques, 55, and a virus inactivation level of -1.15.

In the present invention, as evident from Examples 7 and 8, a viral proliferation inhibitory effect can be obtained even without (2S,3S)-2,3-butanediol, as long as the amount of (meso)-2,3-butanediol is equal to or less than 0.13 times, particularly equal to or less than 0.05 times, the amount of (2R,3R)-2,3-butanediol in terms of weight ratio.

As a result of a comparative moisture-retention test performed on (meso)-2,3-butanediol, (2R,3R)-2,3-butanediol, and (2S,3S)-2,3-butanediol in accordance with the moisture-retention test described in JP 2002-212021 A, which is incorporated herein by reference, it was found that the moisture-retention property of (meso)-2,3-butanediol is higher than those of (2R,3R)-2,3-butanediol and (2S,3S)-2,3-butanediol.

As described above, the composition of the present invention has an excellent viral proliferation inhibitory effect, and can be used for applications in which a viral proliferation inhibitory effect is required, such as in cosmetic raw materials.

**Claims**

1.  A composition, comprising

at least (2R,3R)-2,3-butanediol and/or (2S,3S)-2,3-butanediol and (meso)-2,3-butanediol,
wherein in terms of weight ratio, an amount of the (meso)-2,3-butanediol is equal to or less than twice a combined amount of the (2R,3R)-2,3-butanediol and the (2S,3S)-2,3-butanediol.

2. The composition according to claim 1,
wherein in terms of weight ratio, the amount of the (meso)-2,3-butanediol is equal to or less than 1.5 times the combined amount of the (2R,3R)-2,3-butanediol and the (2S,3S)-2,3-butanediol.

3. The composition according to claim 1,
wherein in terms of weight ratio, the amount of the (meso)-2,3-butanediol is equal to or less than 0.5 times the combined amount of the (2R,3R)-2,3-butanediol and the (2S,3S)-2,3-butanediol.

4. The composition according to claim 1,
wherein in terms of weight ratio, the amount of the (meso)-2,3-butanediol is equal to or less than 0.13 times the combined amount of the (2R,3R)-2,3-butanediol and the (2S,3S)-2,3-butanediol.

5. The composition according to claim 1,
wherein in terms of weight ratio, the amount of the (meso)-2,3-butanediol is equal to or less than 0.05 times the combined amount of the (2R,3R)-2,3-butanediol and the (2S,3S)-2,3-butanediol.

6. The composition according to claim 1,

wherein the composition comprises the (2S,3S)-2,3-butanediol, and
the amount of the (2S,3S)-2,3-butanediol is greater than the amount of the (meso)-2,3-butanediol.

7. The composition according to claim 1,

wherein the composition comprises the (2R,3R)-2,3-butanediol, and
the amount of the (2R,3R)-2,3-butanediol is greater than the amount of the (meso)-2,3-butanediol.

8. The composition according to claim 1,

wherein the composition comprises both the (2S,3S)-2,3-butanediol and the (2R,3R)-2,3-butanediol, and
the amount of the (2S,3S)-2,3-butanediol is greater than the amount of the (2R,3R)-2,3-butanediol and greater than the amount of the (meso)-2,3-butanediol.

9. The composition according to claim 1,

wherein the composition comprises both the (2S,3S)-2,3-butanediol and the (2R,3R)-2,3-butanediol, and
the amount of the (2R,3R)-2,3-butanediol is greater than the amount of the (2S,3S)-2,3-butanediol and greater than the amount of the (meso)-2,3-butanediol.

10. The composition according to any one of claims 1 to 5 and 7,
wherein the composition is free from (2S,3S)-2,3-butanediol.

11. The composition according to any one of claims 1 to 7,
wherein the composition is free from a racemic mixture of (2R,3R)-2,3-butanediol and (2S,3S)-2,3-butanediol.

12. The composition according to any one of claims 1 to 11, for use as a viral proliferation inhibitor.

13. A cosmetic raw material composition, comprising the composition according to any one of claims 1 to 12.

FIG.1

FIG.2

## INTERNATIONAL SEARCH REPORT

| International application No. |
|---|
| **PCT/JP2024/025113** |

| | |
|---|---|
| **A. CLASSIFICATION OF SUBJECT MATTER** | |

*A01N 31/02*(2006.01)i; *A01P 1/00*(2006.01)i; *A61K 8/34*(2006.01)i; *A61Q 1/00*(2006.01)i; *A61Q 19/00*(2006.01)i
FI: A01N31/02; A01P1/00; A61K8/34; A61Q1/00; A61Q19/00

According to International Patent Classification (IPC) or to both national classification and IPC

| | |
|---|---|
| **B. FIELDS SEARCHED** | |

Minimum documentation searched (classification system followed by classification symbols)

A01N31/02; A01P1/00; A61K8/34; A61Q1/00; A61Q19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KR 10-2019-0141515 A (INDUSTRY FOUNDATION OF CHONNAM NATIONAL UNIVERSITY) 24 December 2019 (2019-12-24) paragraphs [0030]-[0036], [0064] | 1-11 |
| A | | 12-13 |
| X | JP 2015-228804 A (MITSUBISHI CHEMICAL CORPORATION) 21 December 2015 (2015-12-21) paragraph [0002], example 5, table 2 | 1-3, 13 |
| A | | 4-12 |
| X | JP 2015-54962 A (RICOH COMPANY, LTD.) 23 March 2015 (2015-03-23) claims, examples 2-18, paragraph [0010] | 1-11 |
| A | | 12-13 |
| X | JP 2021-528429 A (GS CALTEX CORP.) 21 October 2021 (2021-10-21) claims, examples 2, 4, paragraph [0022] | 1-6, 11, 13 |
| A | | 7-10, 12 |

| ✓ Further documents are listed in the continuation of Box C. | ✓ See patent family annex. |
|---|---|

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **30 August 2024** | **17 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| International application No. |
| --- |
| **PCT/JP2024/025113** |

### C.  DOCUMENTS CONSIDERED TO BE RELEVANT

| Category* | Citation of document, with indication, where appropriate, of the relevant passages | Relevant to claim No. |
| --- | --- | --- |
| X | KONG, H. G. et al. Stereoisomers of the Bacterial Volatile Compound 2,3-Butanediol Differently Elicit Systemic Defense Responses of Pepper against Multiple Viruses in the Field. Frontiers in Plant Science. 22 February 2018, vol. 9, article 90, pp. 1-13 | 1-12 |
| A | abstract, column "MATERIALS AND METHODS", fig. 4 | 13 |
| A | JP 2004-529889 A (KONOWALCHUK, Tomas W.) 30 September 2004 (2004-09-30) entire text | 1-13 |

**INTERNATIONAL SEARCH REPORT**
Information on patent family members

International application No.

**PCT/JP2024/025113**

| Patent document cited in search report | | | Publication date (day/month/year) | Patent family member(s) | | Publication date (day/month/year) |
|---|---|---|---|---|---|---|
| KR | 10-2019-0141515 | A | 24 December 2019 | (Family: none) | | |
| JP | 2015-228804 | A | 21 December 2015 | (Family: none) | | |
| JP | 2015-54962 | A | 23 March 2015 | US 2015/0077479 <br> claims, examples 2-18, <br> paragraph [0010] | A1 | |
| JP | 2021-528429 | A | 21 October 2021 | US 2021/0252423 <br> claims, examples 2, 4, <br> paragraph [0022] <br> WO 2019/245317 <br> EP 3812023 <br> KR 10-2020-0140913 <br> CN 112334205 | A1 <br><br><br> A1 <br> A1 <br> A <br> A | |
| JP | 2004-529889 | A | 30 September 2004 | US 2002/0165277 <br> entire text <br> WO 2002/069887 <br> EP 1372389 <br> CN 1505478 <br> KR 10-2004-0038908 | A1 <br><br> A2 <br> A1 <br> A <br> A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018531991 T
- JP 2017531671 T
- JP H10234390 A
- JP 2015518736 T
- JP 2004357639 A
- JP 2018177747 A
- JP 2002212021 A

**Non-patent literature cited in the description**

- *Reports of the Central Customs Laboratory*, 1980 (20), 123-127
- *CHEMICAL ABSTRACTS*, 5341-95-7